# EUROPEAN PATENT SPECIFICATION

(11) **EP 0 624 569 B1**
(45) Date of publication and mention of the grant of the patent: **29.07.1998**
(21) Application number: 94110261.8
(22) Date of filing: 24.02.1992
(51) Int. Cl.: C07C 229/42, A61K 31/405, A61K 31/19

(54) **NMDA antagonists**
NMDA Antagonisten
Antagonistes du NMDA

(30) Priority: 28.02.1991 US 662670
(43) Date of publication of application: 17.11.1994
(62) Divisional of application: 92103066.4
(73) Proprietor: MERRELL PHARMACEUTICALS INC., Cincinnati, Ohio 45215-6300 (US)
(72) Inventor: Palfreyman, Michael G., CIncinnati, Ohio 45249 (US); Salituro, Francesco G., Fairfield, Ohio 45014 (US); McDonald, Ian A., Loveland, Ohio 45140 (US); Schwarcz, Robert, Baltimore, Maryland 21209 (US)
(74) Representative: VOSSIUS & PARTNER

(56) References cited:
- EP-A- 0 421 946
- WO-A-86/03489
- WO-A-88/09789
- NEUROLOGY vol. 40, no. 4 , April 1990 pages 691 - 695 A.FREESE ET AL 'Kynurenine metabolites of tryptophan: implications for neurologic diseases'
- CHEMICAL ABSTRACTS, vol. 112, no. 15, 9 April 1990, Columbus, Ohio, US; abstract no. 135860g, R. LASKE ET AL 'Investigations on the antiproliferative effects of amino acid antagonists targeting for aminoacyl-tRNA synthetases, Part I' page 413 ; & ARCH. PHARM. vol. 322, no. 12 , 1989 , GERMANY pages 847 - 852
- CHEMICAL ABSTRACTS, vol. 97, no. 15, 1982, Columbus, Ohio, US; abstract no. 125986k, 'Halogenoanthranyloylalanines as sweeteners' page 580 ; & JP-A-8 218 458 (TANABE SEIYAKU CO. LTD.) 16 April 1982
- CHEMICAL ABSTRACTS, vol. 92, no. 5, 1980, Columbus, Ohio, US; abstract no. 36767u, 'The mechanism of kynurenine hydrolysis catalysed kynurenine' page 340 ; & J BIOCHEM. vol. 86, no. 5 , 1979 , TOKYO pages 1199 - 1209 TANIZAWA, K ET AL
- J. MED. CHEM vol. 37, no. 3 , 4 February 1994 pages 334 - 336 F.G.SALITURO ET AL 'Enzyme-activated antagonists of the Strychnine-insensitive Glycine/NMDA Receptor'

## Description

The present invention is directed to a new use for a group of known 6-halo-tryptophan and 4-halo-kynurenine derivatives for the preparation of a pharmaceutical composition as excitatory amino acid antagonists, for use in the treatment of disease states such as epilepsy, anxiety and stroke. In EP-A 421 946 published on April 10, 1991 and designating the contracting states DE, FR and GB, the use of 6-chlorotryptophan as an NMDA antagonist is disclosed. The present invention pertains to the following known compounds which are excitatory amino acid antagonists: 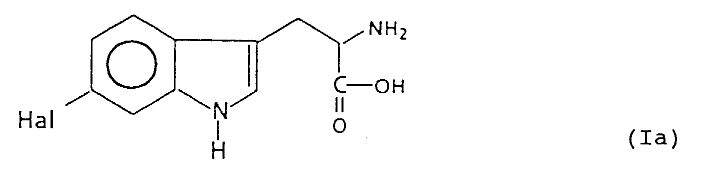 (all contracting states except DE, FR and GB)
and 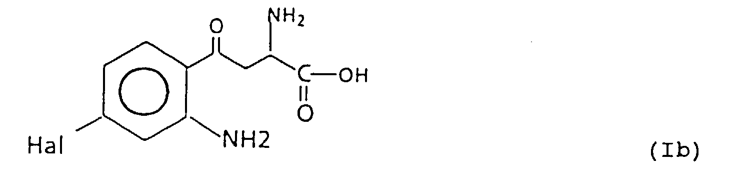 in which Hal is represented by a halogen atom, or a pharmaceutically acceptable salt thereof.

As used in this application:
a) the term "halogen" refers to a fluorine, chlorine, or bromine atom;
b) the term "pharmaceutically acceptable addition salts" refers to either an acid addition or a basic addition salt.

The compounds of Formula Ia and Ib can exist as either pharmaceutically acceptable acid addition salts or as pharmaceutically acceptable basic addition salts. These compounds may also exist as zwitterions.

The expression "pharmaceutically acceptable acid addition salts" is intended to apply to any non-toxic organic or inorganic acid addition salt of the base compounds represented by Formula Ia or Ib or any of its intermediates. Illustrative inorganic acids which form suitable salts include hydrochloric, hydrobromic, sulphuric, and phosphoric acid and acid metal salts such as sodium monohydrogen orthophosphate, and potassium hydrogen sulfate. Illustrative organic acids which form suitable salts include the mono-, di-, and tricarboxylic acids. Illustrative of such acids are for example, acetic, glycolic, lactic, pyruvic, malonic, succinic, glutaric, fumaric, malic, tartaric, citric, ascorbic, maleic, hydroxymaleic, benzoic, hydroxy-benzoic, phenylacetic, cinnamic, salicyclic, 2-phenoxy-benzoic, p-toluenesulfonic acid, and sulfonic acids such as methane sulfonic acid and 2-hydroxyethane sulfonic acid. Such salts can exist in either a hydrated or substantially anhydrous form. In general, the acid addition salts of these compounds are soluble in water and various hydrophilic organic solvents, and which in comparison to their free base forms, generally demonstrate higher melting points.

The expression "pharmaceutically acceptable basic addition salts" is intended to apply to any non-toxic organic or inorganic basic addition salts of the compounds represented by Formula Ia or Ib or any of its intermediates. Illustrative bases which form suitable salts include alkali metal or alkaline-earth metal hydroxides such as sodium, potassium, calcium, magnesium, or barium hydroxides; ammonia, and aliphatic, alicyclic, or aromatic organic amines such as methylamine, dimethylamine, trimethylamine, and picoline. Either the mono- or di-basic salts can be formed with those compounds.

All of the compounds of Formula Ia and Ib contain a chiral center and thus can exist as optical isomers. Any reference to these compounds or their intermediates should be construed as referring to either a racemic mixture or an individual optical isomer. The specific optical isomers can be separated and recovered by techniques known in the art such as chromatography on chiral stationary phases or resolution via chiral salt formation and subsequent separation by selective crystallization. Alternatively utilization of a specific optical isomer as the starting material will produce the corresponding isomer as the final product.

Examples of compounds encompassed by Formula I a include:
6-chloro-tryptophan;
6-fluoro-tryptophan.

Examples of compounds encompassed by Formula 1 b include:
4-chloro-kynurenine;
4-fluoro-kyurenine.

The compounds of Formula Ia and Ib are known in the art. Methods for preparing these compounds are known in the art.

As noted above, the compounds of Formula Ia and Ib (hereinafter "the compounds") antagonize the effects which excitatory amino acids have upon the NMDA receptor complex. This antagonist effect can be demonstrated by their ability to prevent NMDA-stimulated cyclic GMP accumulation in neonatal rat cerebellar tissue. This test is based upon the phenomenon that when samples of neonatal rat cerebellar tissue are exposed to the agonist, NMDA, there is an increase in cyclic GMP levels within this tissue. NMDA antagonists inhibit or decrease this rise in cyclic GMP levels. This test can be performed by methods similar to those of Baron et al., J. Pharmacol. Exp. Ther. Vol 250 page 162 (1989).

The compounds exhibit anti-convulsant properties and pharmaceutical compositions containing them and are useful in the treatment of epilepsy. They are useful in the treatment of grand mal seizures, petit mal seizures, psychomotor seizures, autonomic seizures, etc. One method of demonstrating their anti-epileptic properties is by their ability to inhibit the seizures that are caused by the administration of quinolinic acid, an NMDA agonist. This test can be conducted in the following manner.

One group containing ten mice are administered 0.01 - 100 µg of test compound intracerebroventricularly in a volume of 5 microliters of saline. A second control group containing an equal number of mice are administered an equal volume of saline as a control. Approximately 5 minutes later, both groups are administered 7.7 micrograms of quinolinic acid intracerebroventricularly in a volume of 5 microliters of saline. The animals are observed for 15 minutes thereafter for signs of clonic-tonic seizures. The control group will have a statistically higher rate of clonic-tonic seizures than will the test group.

The compounds are useful for preventing or minimizing the damage which nervous tissues contained within the CNS suffer upon exposure to either ischemic, hypoxic, or hypoglycemic conditions or as the result of physical trauma. Representative examples of such conditions include strokes or cerebrovascular accidents, hyperinsulinemia, cardiac arrest, physical trauma, drownings, suffocation, and neonatal anoxic trauma. The pharmaceutical compositions containing the compounds should be administered to the patient within 24 hours of the onset of the hypoxic, ischemic, or hypoglycemic condition in order for the compounds to effectively minimize the CNS damage which the patient will experience.

The pharmaceutical compositions containing the compounds are also useful in the treatment of neurodegenerative diseases such as Huntington's disease, Alzheimer's disease, senile dementia, glutaric acidaemia type I, Parkinson's disease, multi-infarct dementia, and neuronal damage associated with uncontrolled seizures. The administration of these compounds to a patient experiencing such a condition will serve to either prevent the patient from experiencing further neurodegeneration or it will decrease the rate at which the neurodegeneration occurs.

As is apparent to those skilled in the art, the compounds will not correct any CNS damage that has already occurred as the result of either disease, or a lack of oxygen or sugar. As used in this application, the term "treat" refers to the ability of the compounds to prevent further damage or delay the rate at which any further damage occurs.

The compounds exhibit an anxiolytic effect and pharmaceutical compositions containing them are thus useful in the treatment of anxiety. The compounds also exhibit an analgesic effect and the pharmaceutical compositions containing them are useful in controlling pain. They may be co-administered with a narcotic analgesic such as morphine, demerol, etc. In addition to lowering the dose of narcotic required, a decrease in the rate at which patients develop tolerance to the pharmacological effects of these narcotics is achieved. It is also believed that this co-administration will help to prevent the patient from becoming addicted to the narcotic.

They are also effective in the treatment of migraine. They can be used prophylactically or to relieve the symptoms associated with a migraine episode.

In order to exhibit these therapeutic properties, pharmaceutical compositions containing the compounds need to be administered in a quantity sufficient to inhibit the effect which the excitatory amino acids have upon the NMDA receptor complex. The dosage range at which these compounds exhibit this antagonistic effect can vary widely depending upon the particular disease being treated, the severity of the patient's disease, the patient, the particular compound being administered, the route of administration, and the presence of other underlying disease states within the patient, etc. Typically the compounds exhibit their therapeutic effect at a dosage range of from about 0.1 mg/kg/day to about 100 mg/kg/day for any of the diseases or conditions listed above. Repetitive daily administration may be desirable and will vary according to the conditions outlined above.

It has been discovered that probenecid will potentiate the therapeutic activity of the excitatory amino acid antagonists of the present invention. Thus the compounds will exhibit their therapeutic effects at lower doses and for longer periods in patients who are concurrently receiving probenecid. The mechanism by which probenecid potentiates their effects is not fully understood, however it is believed that probenecid decreases the rate at which the compounds are removed from the central nervous system as well as decreasing the rate of excretion by the kidneys. Probenecid increases the effective concentration of these compounds in both the CNS and in the systemic circulation.

Probenecid is known in the art. It is available commercially from Merck Sharp and Dohme under the tradename Benemid® as well as being available from numerous other sources. Probenecid is a uricosuric agent and is utilized in the treatment of gout. Probenecid is a renal tubular transport blocking agent and has been utilized to increase plasma levels of penicillin. The pharmacology of probenecid is described in detail in the 45th Edition of the Physicians Desk reference on page 1379. Probenecid is currently available commercially as tablets. The sodium salt of probenecid is readily water soluble and injectable dosage form can be prepared from this salt using techniques well known to those skilled in the art.

The pharmaceutical compositions containing the compounds of the invention may be administered concurrently with probenecid in order to treat any of the diseases or conditions described above. The quantity of probenecid that is required to potentiate the therapeutic effects of the compounds can vary widely depending upon the particular compound being administered, the patient, and the presence of other underlying disease states within the patient, etc. Typically though, the probenecid may be administered at a dosage of from 0.5-3g/day. Repetitive daily administration may be desirable and will vary according to the conditions outlined above. The probenecid will typically be administered from 2-4 times daily.

With the concurrent administration of probenecid, the dosage range for the excitatory amino antagonists may be adjusted lower by a factor of from 2-10. Alternatively, the compounds of Formulae Ia or Ib may be administered at the same dosage range in order to obtain an enhanced effect due to the higher therapeutic concentrations obtained.

The pharmaceutical compositions containing the compounds of the present invention may be administered by a variety of routes. They are effective if administered orally. They may also be administered parenterally (i.e. subcutaneously, intravenously, intramuscularly, intraperitoneally, or intrathecally).

Pharmaceutical compositions can be manufactured utilizing techniques known in the art. Typically an antagonistic amount of the compound will be admixed with a pharmaceutically acceptable carrier.

For oral administration, the compounds can be formulated into solid or liquid preparations such as capsules, pills, tablets, lozenges, melts, powders, suspensions, or emulsions. Solid unit dosage forms can be capsules of the ordinary gelatin type containing, for example, surfactants, lubricants and inert fillers such as lactose, sucrose, and cornstarch or they can be sustained release preparations.

In another embodiment, the compounds can be tableted with conventional tablet bases such as lactose, sucrose, and cornstarch in combination with binders, such as acacia, cornstarch, or gelatin, disintegrating agents such as potato starch or alginic acid, and a lubricant such as stearic acid or magnesium stearate. Liquid preparations are prepared by dissolving the active ingredient in an aqueous or non-aqueous pharmaceutically acceptable solvent which may also contain suspending agents, sweetening agents, flavoring agents, and preservative agents as are known in the art.

For parenteral administration the compounds may be dissolved in a physiologically acceptable pharmaceutical carrier and administered as either a solution or a suspension. Illustrative of suitable pharmaceutical carriers are water, saline, dextrose solutions, fructose solutions, ethanol, or oils of animal, vegetative, or synthetic origin. The pharmaceutical carrier may also contain preservatives, buffers, etc., as are known in the art. When the compounds are being administered intrathecally, they may also be dissolved in cerebrospinal fluid as is known in the art.

The compounds of Formulae Ia and Ib and the probenecid can be administered as two different pharmaceutical dosage forms. Alternatively, in order to increase patient convenience, the compounds and the probenecid may be compounded into a single pharmaceutical dosage form. These pharmaceutical compositions can be manufactured utilizing techniques known in the art similar to those described above. Typically an antagonistic amount of the compound of Formula I and an effective amount of probenecid will be admixed with a pharmaceutically acceptable carrier.

As used in this application:
a) the term "patient" refers to warm blooded animals such as, for example, guinea pigs, mice, rats, cats, rabbits, dogs, monkeys, chimpanzees, and humans;
b) the term "treat" refers to the ability of the compounds to either relieve, alleviate, or slow the progression of the patient's disease or prophylactically prevent its occurrence or the manifestation of its symptoms;
c) the phrase "antagonize the effects of excitatory amino acids" and the phrase "excitatory amino acid antagonist" should be referred to the ability of the compounds to inhibit or decrease the rate at which glutamate or glycine produce neurotransmission at the NMDA receptor complex, and;
d) the term "neurodegeneration" refers to a progressive death and disappearance of a population of nerve cells occurring in a manner characteristic of a particular disease state and leading to brain damage;
e) the phrase "concurrent administration" refers to administering the probenecid at an appropriate time so that it will potentiate the antagonistic effects of the compounds of Formula Ia or Ib. This may means simultaneous administration or administration at appropriate but different times. Establishing such a proper dosing schedule will be readily apparent to one skilled in the art.

The compounds may also be admixed with any inert carrier and utilized in laboratory assays in order to determine the concentration of the compounds within the serum, urine, etc., of the patient as is known in the art.

Neurodegenerative diseases are typically associated with a dysfunction of NMDA receptors. Thus, the compounds of Formula I may be utilized in diagnostic procedures to aid physicians with the diagnosis of neurodegenerative diseases. The compounds may be labeled with imaging agents known in the art such as isotopic atoms and administered to a patient in order to determine whether the patient is exhibiting a decreased number of NMDA receptors and the rate at which that loss is occurring.

## Claims (Claims for the following Contracting State(s): AT, BE, CH, LI, DK, ES, GR, IT, LU, MC, NL, PT, SE)

1. Use of a compound of the formula 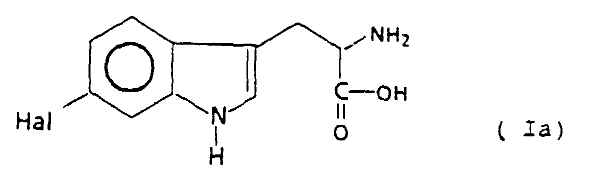 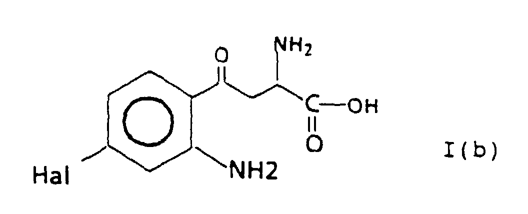 in which Hal is represented by a halogen atom, or a pharmaceutically acceptable salt thereof, for the preparation of a pharmaceutical composition useful in antagonising the effects of excitatory amino acids upon the NMDA receptor complex.

2. Use of a compound as defined in claim 1 for the preparation of a pharmaceutical composition useful in the treatment of epilepsy.

3. Use of a compound as defined in claim 1 for the preparation of a pharmaceutical composition useful in the treatment of neurodegenerative diseases.

4. Use of a compound as defined in claim 1 for the preparation of a pharmaceutical composition useful in preventing ischemic/hypoxic/hypoglycemic damage to cerebral tissue.

5. Use of a compound as defined in claim 1 for the preparation of a pharmaceutical composition useful in the treatment of anxiety.

6. Use of a compound as defined in claim 1 for the preparation of a pharmaceutical composition useful in producing an analgesic effect.

7. Use of a compound as defined in claim 1 for the preparation of a pharmaceutical composition useful in the treatment of migraine.

8. A pharmaceutical composition comprising a compound as defined in claim 1 in admixture with probenecid.

9. A pharmaceutical composition comprising a compound as defined in claim 1 and probenecid in two separate dosage forms for concurrent administration.

## Claims (Claims for the following Contracting State(s): DE, FR, GB)

1. Use of a compound of the formula 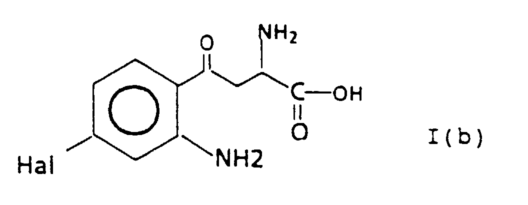 in which Hal is represented by a halogen atom, or a pharmaceutically acceptable salt thereof, for the preparation of a pharmaceutical composition useful in antagonising the effects of excitatory amino acids upon the NMDA receptor complex.

2. Use of a compound as defined in claim 1 for the preparation of a pharmaceutical composition useful in the treatment of epilepsy.

3. Use of a compound as defined in claim 1 for the preparation of a pharmaceutical composition useful in the treatment of neurodegenerative diseases.

4. Use of a compound as defined in claim 1 for the preparation of a pharmaceutical composition useful in preventing ischemic/hypoxic/hypoglycemic damage to cerebral tissue.

5. Use of a compound as defined in claim 1 for the preparation of a pharmaceutical composition useful in the treatment of anxiety.

6. Use of a compound as defined in claim 1 for the preparation of a pharmaceutical composition useful in producing an analgesic effect.

7. Use of a compound as defined in claim 1 for the preparation of a pharmaceutical composition useful in the treatment of migraine.

8. A pharmaceutical composition comprising a compound as defined in claim 1 in admixture with probenecid.

9. A pharmaceutical composition comprising a compound as defined in claim 1 and probenecid in two separate dosage forms for concurrent administration.

## Patentansprüche (Patentansprüche für folgende(n) Vertragsstaat(en): AT, BE, CH, LI, DK, ES, GR, IT, LU, MC, NL, PT, SE)

1. Verwendung einer Verbindung der Formel 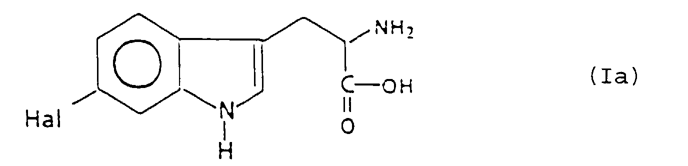 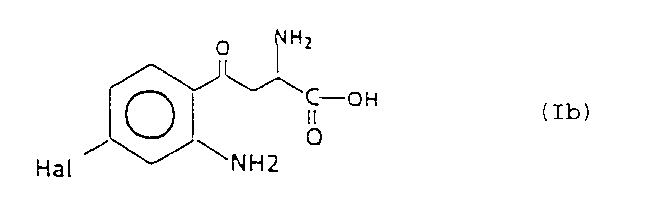 in der Hal durch ein Halogenatom wiedergegeben wird, oder eines pharmazeutisch verträglichen Salzes davon, zur Herstellung eines Arzneimittels, als Antagonist exzitatorischer Aminosäuren am NMDA-Rezeptorkomplex.

2. Verwendung einer Verbindung nach Anspruch 1 zur Herstellung eines Arzneimittels, das zur Behandlung von Epilepsie geeignet ist.

3. Verwendung einer Verbindung nach Anspruch 1 zur Herstellung eines Arzneimittels, das zur Behandlung von neurodegenerativen Erkrankungen geeignet ist.

4. Verwendung einer Verbindung nach Anspruch 1 zur Herstellung eines Arzneimittels, das zur Verhinderung von ischämischer/hypoxischer/hypoglykämischer Schädigung von Hirngewebe geeignet ist.

5. Verwendung einer Verbindung nach Anspruch 1 zur Herstellung eines Arzneimittels, das zur Behandlung von Angstzuständen geeignet ist.

6. Verwendung einer Verbindung nach Anspruch 1 zur Herstellung eines Arzneimittels, das zur Erzeugung einer analgetischen Wirkung geeignet ist.

7. Verwendung einer Verbindung nach Anspruch 1 zur Herstellung eines Arzneimittels, das zur Behandlung von Migräne geeignet ist.

8. Arzneimittel, umfassend eine Verbindung nach Anspruch 1, in Anmischung mit Probenecid.

9. Arzneimittel, umfassend eine Verbindung nach Anspruch 1 und Probenecid in zwei gesonderten Dosierungsformen zur gleichzeitigen Verabreichung.

## Patentansprüche (Patentansprüche für folgende(n) Vertragsstaat(en): DE, FR, GB)

1. Verwendung einer Verbindung der Formel 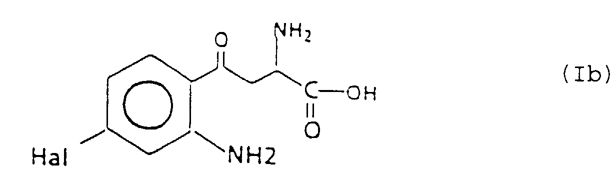 in der Hal durch ein Halogenatom wiedergegeben wird, oder eines pharmazeutisch verträglichen Salzes davon, zur Herstellung eines Arzneimittels, als Antagonist exzitatorischer Aminosäuren am NMDA-Rezeptorkomplex.

2. Verwendung einer Verbindung nach Anspruch 1 zur Herstellung eines Arzneimittels, das zur Behandlung von Epilepsie geeignet ist.

3. Verwendung einer Verbindung nach Anspruch 1 zur Herstellung eines Arzneimittels, das zur Behandlung von neurodegenerativen Erkrankungen geeignet ist.

4. Verwendung einer Verbindung nach Anspruch 1 zur Herstellung eines Arzneimittels, das zur Verhinderung von ischämischer/hypoxischer/hypoglykämischer Schädigung von Hirngewebe geeignet ist.

5. Verwendung einer Verbindung nach Anspruch 1 zur Herstellung eines Arzneimittels, das zur Behandlung von Angstzuständen geeignet ist.

6. Verwendung einer Verbindung nach Anspruch 1 zur Herstellung eines Arzneimittels, das zur Erzeugung einer analgetischen Wirkung geeignet ist.

7. Verwendung einer Verbindung nach Anspruch 1 zur Herstellung eines Arzneimittels, das zur Behandlung von Migräne geeignet ist.

8. Arzneimittel, umfassend eine Verbindung nach Anspruch 1, in Anmischung mit Probenecid.

9. Arzneimittel, umfassend eine Verbindung nach Anspruch 1 und Probenecid in zwei gesonderten Dosierungsformen zur gleichzeitigen Verabreichung.

## Revendications (Revendications pour l'(les) Etat(s) contractant(s) suivant(s): AT, BE, CH, LI, DK, ES, GR, IT, LU, MC, NL, PT, SE)

1. Utilisation d'un composé de formule 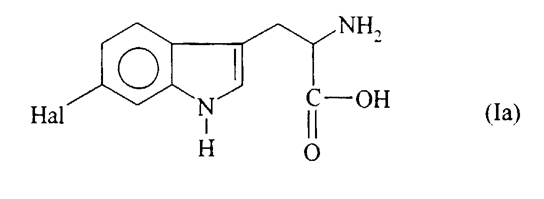 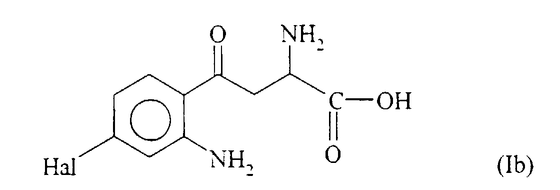 où Hal est représenté par un atome d'halogène, ou d'un sel pharmaceutiquement acceptable de celui-ci. pour la préparation d'une composition pharmaceutique utile pour antagoniser les effets des acides aminés excitateurs sur le complexe des récepteurs de NMDA.

2. Utilisation d'un composé tel que défini dans la revendication 1 pour la préparation d'une composition pharmaceutique utile dans le traitement de l'épilepsie.

3. Utilisation d'un composé tel que défini dans la revendication 1 pour la préparation d'une composition pharmaceutique utile dans le traitement des maladies neurodégénératives.

4. Utilisation d'un composé tel que défini dans la revendication 1 pour la préparation d'une composition pharmaceutique utile dans la prévention d'une lésion ischémique/hypoxique/hypoglycémique du tissu cérébral.

5. Utilisation d'un composé tel que défini dans la revendication 1 pour la préparation d'une composition pharmaceutique utile dans le traitement de l'anxiété.

6. Utilisation d'un composé tel que défini dans la revendication 1 pour la préparation d'une composition pharmaceutique utile dans la production d'un effet analgésique.

7. Utilisation d'un composé tel que défini dans la revendication 1 pour la préparation d'une composition pharmaceutique utile dans le traitement de la migraine.

8. Composition pharmaceutique comprenant un composé tel que défini dans la revendication 1 en mélange avec du probénécide.

9. Composition pharmaceutique comprenant un composé tel que défini dans la revendication 1 et du probénécide dans deux formes galéniques séparées pour l'administration conjointe.

## Revendications (Revendications pour l'(les) Etat(s) contractant(s) suivant(s): DE, FR, GB)

1. Utilisation d'un composé de formule 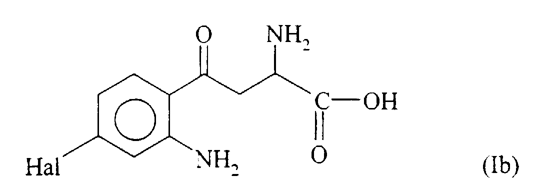 où Hal est représenté par un atome d'halogène, ou d'un sel pharmaceutiquement acceptable de celui-ci. pour la préparation d'une composition pharmaceutique utile pour antagoniser les effets des acides aminés excitateurs sur le complexe des récepteurs de NMDA.

2. Utilisation d'un composé tel que défini dans la revendication 1 pour la préparation d'une composition pharmaceutique utile dans le traitement de l'épilepsie.

3. Utilisation d'un composé tel que défini dans la revendication 1 pour la préparation d'une composition pharmaceutique utile dans le traitement des maladies neurodégénératives.

4. Utilisation d'un composé tel que défini dans la revendication 1 pour la préparation d'une composition pharmaceutique utile dans la prévention d'une lésion ischémique/hypoxique/hypoglycémique du tissu cérébral.

5. Utilisation d'un composé tel que défini dans la revendication 1 pour la préparation d'une composition pharmaceutique utile dans le traitement de l'anxiété.

6. Utilisation d'un composé tel que défini dans la revendication 1 pour la préparation d'une composition pharmaceutique utile dans la production d'un effet analgésique.

7. Utilisation d'un composé tel que défini dans la revendication 1 pour la préparation d'une composition pharmaceutique utile dans le traitement de la migraine.

8. Composition pharmaceutique comprenant un composé tel que défini dans la revendication 1 en mélange avec du probénécide.

9. Composition pharmaceutique comprenant un composé tel que défini dans la revendication 1 et du probénécide dans deux formes galéniques séparées pour l'administration conjointe.
